# EUROPEAN PATENT APPLICATION

(11) **EP 4 101 425 A1**
(43) Date of publication of application: **14.12.2022**
(21) Application number: 21382510.2
(22) Date of filing: 08.06.2021
(51) Int. Cl.: A61F 5/455

(54) **MENSTRUAL CUP WITH PRODUCT FOR VAGINAL RELEASE**

(71) Applicant: Ecareyou Innovation, S.L., 08173 Sant Cugat del Vallès (ES)
(72) Inventor: GARRIGA I RODO, Joan, 08173 SANT CUGAT DEL VALLÈS (ES)
(74) Representative: Espiell Gomez, Ignacio

(57) **Abstract**

Menstrual cup with vaginal release product, formed from a glass-shaped or "cup" body (2) having a threadlike end (3) to make its withdrawal easy, **characterized in that** the incorporation on at least the external surface of the said body (2) that remains in contact with the vagina wall when the cup (1) is used, of one or more vaginal release products. The product incorporated in the cup (1) is incorporated in a strip (4) impregnated with it and adapted to be adherable on the external surface of the body (2) of the cup (1). Said strip (4) is of 100% soluble material. External surface of the body (2) of the cup (1) possesses a band (5) having a slight offset and said body (2) incorporates a rough area (6).

## Description

### OBJECT DE LA INVENTION

The invention, as stated in the title of this specification, refers to a menstrual cup with product of vaginal release such as a product to protect the vaginal Ph, that contributes, to the function to which it is designed, with advantages and characteristics that will be disclosed in detail thereafter, that mean an improvement to the current state-of-art.

The object of this invention refers to a menstrual cup of the type used to hold back bleeding during the menstruation, that is distinguished in that it incorporates a product of vaginal release such as dilutable agents to help the vaginal wall Ph balance preventing eventual infections provoked by its decrease during the period, said agents being either incorporated in a strip adhered to the cup or incorporated on the own external and rough surface of the cup or integrated in the own material of the cup.

### FIELD OF APPLICATION OF THE INVENTION

The field of application of this invention is within the sector of the pharmaceutical industry, particularly focusing on the scope of the articles for personal hygiene and at same time, to the products of vaginal release.

### BACKGROUND OF THE INVENTION

In certain occasions, it is necessary to apply products within the vagina, that means vaginal release products.

For example, at the moment when the menstruation, it happens that the vagina has a decrease of Ph becoming more alkaline. Thus, the bacteria of the vaginal flora die, because they are in a hostile environment and they leave a door open to pathogens, either more resistant bacteria (as those causing the toxic shock) that are located in the skin of the vagina entrance or others or fungi, etc. Many women suffer recurrent infections, many times created by this impossibility to compensate the pH during the period.

Until now, this unprotection uses to be treated with vaginal ovules that contain pre and prebiotics, that are administered before and after the menstruation (one day before and one day after), because during its presence, the own blood drags them out of the vagina. Thus, it is tried to have an open window the less possible time.

On its hand, the menstrual cup, also known as vaginal cup is a container similar to a cup, normally of silicone, that is inserted in the vagina during the menstruation to collect the bleeding.

The objective of the invention is, therefore, to prevent the above-mentioned problems by means of using the menstrual cup as a means to dispense products such as the said prebiotics, taking advantage of its contact with the vaginal wall during the period without bleeding occurs.

On the other hand, and as reference to the current state-of-the-art, at least the applicant is not aware of the existence of any other menstrual cup or any other invention of similar vaginal application, that possesses technical and structural characteristics equal or similar to those the present possesses that are claimed herein.

### EXPLANATION OF THE INVENTION

The menstrual cup with vaginal release product as a product to protect the vaginal Ph that the invention proposes is configured as the suitable solution to the above-mentioned objective, the characterizing details making it possible and distinguishing it duly appearing in the final claims attached to this description.

Concretely, what is proposed by the invention, as above stated, is a menstrual cup of the type used to hold back bleeding during the menstruation, that possesses the advantageous feature of incorporating a vaginal release product such as dilutable agents that help to maintain the vaginal wall Ph balance during the menstruation, preventing eventual infections provoked by its decrease during the days the period lasts.

For this, in a first variant of embodiment, the said vaginal release product such as the agents are incorporated in a strip adherable on the cup external surface.

In a preferred embodiment, the said strip is a strip or "band aid", 100% soluble that contains the vaginal release product to be released as the elements to be able to help to balance the vagina Ph, as well as to provide a healthy environment and prone to the development of the vaginal flora, the great natural barrier against the entrance of pathogens. The band aid is placed in the bowl of the menstrual cup, trying to provide the maxim contact with the flora wall and, this way, at the moment of the menstruation be able to intervene in the flora and in its PH. The blood of the menstruation is collected within the cup; therefore, its external surface never stops being in contact with the vagina walls, without interferences with the flora mucosa, helping to create an optimum environment for its preservation.

The use of the cup lasts 8 to 12 hours, therefore any active principle can be administered in a continuous and controlled manner and, depending on the speed sought, it can be more or less soluble..more or less quick.

As it was commented, sometimes, as the surface of the cup once it is placed is kept in direct contact with the flora, the agents can also be supplied with the said principles through oils or any other element that is compatible with the silicone of the cup, as well as create some physical modification of the cup, such as its roughness, for a best adherence of the substances having a different molecular weight to the cup.

For this, and to provide the said best contact, in a preferred embodiment, the drawing of the cup wall has been modified in order that placing the adherable strip is more stable, without creating folds or tips that could damage the vagina internal part.

For this, the area of the external surface of the cup where the band aid is placed possesses a slight offset of the band aid thickness and in addition it is lacking any engraving in texture making that it is fully smooth and therefore easier to adhere with water to the band aid.

This option has the advantage that the cup can be re-used simply by incorporating a new band aid in every use, which can be commercialized to that effect independently from the own cup.

In a second variant of embodiment of the menstrual cup object of the invention, instead of incorporating an adherable strip, it is the own cup, on the external surface of its wall that possesses roughness designed so that it allows to hold back the vaginal release product such as the protecting agent, either in liquid form or gel, with the protobiotic that maintains the Ph balance while it is absorbed by the body.

In this case, the cup can be re-used with or without vaginal release product such as the protecting agent and, if applicable, the said vaginal release product will have to be reincorporated.

And, in a third variant of embodiment, it is the own cup that is made of a material that integrates the vaginal release product such as the protecting agent or prebiotic and that is being gradually released, as it is done by a contraceptive ring. In this case, the cup will be for a single use.

### DESCRIPTION OF THE DRAWINGS

To complement the description being carried out and in order to assist to best understand the characteristics of the invention, attached to this specification, as an integral part thereof, is a drawing in which, for illustration and no limitation purpose, the following has been represented:
The figure number 1.- It shows a schematic view in perspective of an example of embodiment of the menstrual cup with vaginal release product such as a product to protect the vaginal Ph in its variant with impregnated adherable strip to its surface; and
the figure number 2.- It shows a schematic view in perspective of an example of embodiment of the menstrual cup with vaginal release product such as a product to protect the vaginal Ph in its variant with impregnable rough surface.

### PREFERRED EMBODIMENT OF THE INVENTION

Seen the figures disclosed, and according to the numerals adopted in them, no limiting two examples of the menstrual cup with vaginal release product such as a product to protect the vaginal Ph of the invention can be seen, which comprises what is disclosed in detail below.

Thus, the cup (1) of the invention, constituted in a well-known manner from a glass-shaped or "cup" body (2) having a threadlike end (3) to make its withdrawal easy, is essentially distinguished because it comprises the incorporation of at least the external surface of the said body (2) that remains in contact with the vagina wall when it is used, of one or more vaginal releasing products such as dilutable agents , for example in the form of probiotics, that help to maintain the vaginal wall Ph balance thus the said surface remains in contact when the cup (1) is used during the menstruation.

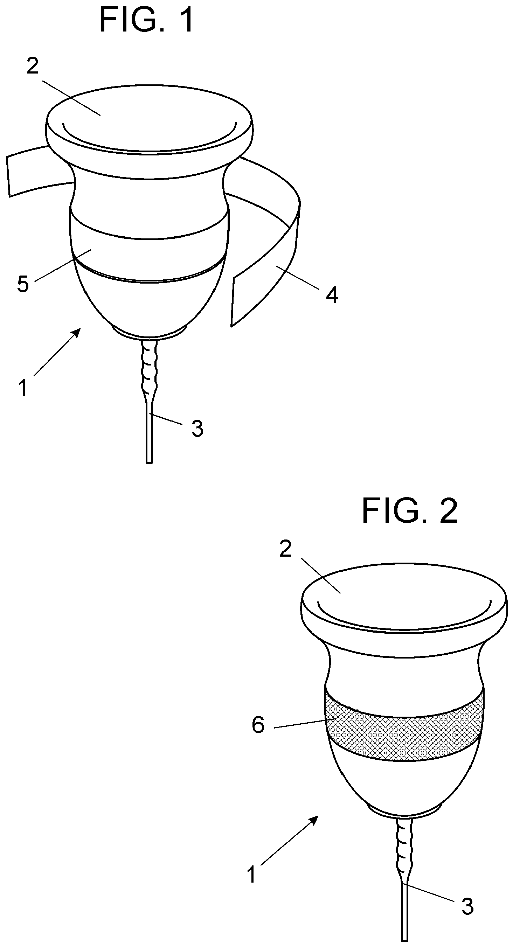

In a first variant of embodiment, as it can be seen in the figure 1, the vaginal release product incorporated in the cup (1) is incorporated in a strip (4) impregnated with it and adapted to be adherable on the external surface of the body (2) of the cup (1)

Preferably, the said strip (4) is of a 100% soluble material.

Preferably, the external surface of the body (2) of the cup (1) possesses a band (5) having a slight offset, of the thickness of the strip (4) disclosed, in which it is lacking any engraving in texture so that its said surface is fully smooth, being designed to make the adherence to the strip (4) easier.

In a second variant of embodiment of the menstrual cup (1) that can be seen in the figure 2, the body (2) of the cup (1) incorporates, on the external surface of the wall of the said body (2), a rough area (6) adapted to hold back the product of vaginal release such as the dilutable agent or agents, for example in the form of probiotics, that help to maintain the vaginal Ph balance, either in liquid form or gel.

And in a third variant of embodiment (not represented), at least the body (2) of the cup (1) is made of a material that integrates vaginal release product such as the dilutable agent or agents, for example in the form of probiotics, that help to maintain the vaginal Ph balance, so that it is gradually released.

Sufficiently disclosed the nature of this invention, as well as the manner of implementing it, it is not deemed necessary to extend any more its explanation in order that any man skilled in the art understands its scope and the advantages arising from it.

## Claims

1. Menstrual cup with vaginal release product, formed from a glass-shaped or "cup" body (2) having a threadlike end (3) to make its withdrawal easy, **characterized in that** the incorporation on at least the external surface of the said body (2) that remains in contact with the vagina wall when the cup (1) is used, of one or more vaginal release products

2. Menstrual cup with vaginal release product, according to the claim 1, **characterized in that** the vaginal release product incorporated in the cup (1) is incorporated in a strip (4) impregnated with it and adapted to be adherable on the external surface of the body (2) of the cup (1)

3. Menstrual cup with vaginal release product according to the claim 2, **characterized in that** the said strip (4) is of 100% soluble material.

4. Menstrual cup with vaginal release product, according to any of the claim 1 or 2, **characterized in that** the external surface of the body (2) of the cup (1) possesses a band (5) having a slight offset, of the thickness of the strip (4) disclosed, and having a smooth surface designed to make the adherence to the strip (4) easy.

5. Menstrual cup with vaginal release product according to the claim 1, **characterized in that** the body (2) of the cup (1) incorporates, on the external surface of the wall of the said body (2), a rough area (6) adapted to hold back the vaginal release product either in liquid form or gel.

6. Menstrual cup with vaginal release product, according to the claim 1, **characterized in that** at least the body (2) of the cup (1) is made of a material that integrates vaginal release product, so that it is gradually released.

7. Menstrual cup with vaginal release product, according to any of the claims 1 to 6, **characterized in that** the vaginal release product is a dilutable agent or agents that help to maintain the vaginal wall Ph balance.

8. Menstrual cup with vaginal release product, according to any of the claims 1 to 7, **characterized in that**, the product is a probiotic agent.

## Amended claims

### Amended claims in accordance with Rule 137(2) EPC.

1. Menstrual cup with vaginal release product, comprising:
• a glass-shaped or "cup" body (2) having a threadlike end (3) to make its withdrawal easy,
• a vaginal release product incorporated on at least the external surface of the said body (2) such that the vaginal release product remains in contact with the vagina wall when the cup (1) is used
**characterized in that** the vaginal release product is impregnated in a strip (4) made of 100% soluble material, the strip (4) adherable on the external surface of the body (2) of the cup (1)

2. Menstrual cup with vaginal release product, according to claim 1 **characterized in that** the external surface of the body (2) of the cup (1) possesses a band (5) having a slight offset, of the thickness of the strip (4) disclosed, and having a smooth surface designed to make the adherence to the strip (4) easy.

3. Menstrual cup with vaginal release product, according to any of the preceding claims , **characterized in that** the vaginal release product is a dilutable agent or agents that help to maintain the vaginal wall Ph balance.

4. Menstrual cup with vaginal release product, according to any of the preceding claims, **characterized in that,** the product is a probiotic agent.
